# EUROPÄISCHE PATENTSCHRIFT

(11) **EP 3 252 490 B1**
(45) Veröffentlichungstag und Bekanntmachung des Hinweises auf die Patenterteilung: **16.09.2020**
(21) Anmeldenummer: 16172882.9
(22) Anmeldetag: 03.06.2016
(51) Int. Cl.: G01R 33/36

(54) **VORRICHTUNG UND VERFAHREN ZUR ÜBERTRAGUNG VON RADIOFREQUENZ-SIGNALEN ÜBER EINE GESCHIRMTE, SYMMETRISCHE LEITUNG**
DEVICE AND METHOD FOR TRANSPORTING RADIOFREQUENCY-SIGNALS THROUGH A SHIELDED, BALANCED LINE
DISPOSITIF ET PROCEDE DE TRANSMISSION DE SIGNAUX RADIOFREQUENCES PAR L'INTERMEDIAIRE D'UNE LIGNE SYMETRIQUE ET BLINDEE

(43) Veröffentlichungstag der Anmeldung: 06.12.2017
(73) Patentinhaber: Siemens Healthcare GmbH, 91052 Erlangen (DE)
(72) Erfinder: BOLLENBECK, Jan, 91330 Eggolsheim (DE)

(56) Entgegenhaltungen:
- WO-A1-2012/003211
- US-A1- 2011 215 807
- US-A1- 2012 106 657

## Beschreibung

Die Erfindung betrifft eine Übertragungsvorrichtung zum Übertragen zweier Hochfrequenzsignale sowie einen Magnetresonanztomographen mit einer erfindungsgemäßen Übertragungsvorrichtung.

Magnetresonanztomographen sind bildgebende Vorrichtungen, die zur Abbildung eines Untersuchungsobjektes Kernspins des Untersuchungsobjektes mit einem starken äußeren Magnetfeld ausrichten und durch ein magnetisches Wechselfeld zur Präzession um diese Ausrichtung anregen. Die Präzession bzw. Rückkehr der Spins aus diesem angeregten in einen Zustand mit geringerer Energie wiederum erzeugt als Antwort ein magnetisches Wechselfeld, auch als Magnetresonanzsignal bezeichnet, das über Antennen empfangen wird.

Mit Hilfe von magnetischen Gradientenfeldern wird den Signalen eine Ortskodierung aufgeprägt, die nachfolgend eine Zuordnung von dem empfangenen Signal zu einem Volumenelement ermöglicht. Das empfangene Signal wird dann ausgewertet und eine dreidimensionale bildgebende Darstellung des Untersuchungsobjektes bereitgestellt.

Zur Anregung der Präzession der Spins sind magnetische Wechselfelder mit einer Frequenz, die der Larmorfrequenz bei der jeweiligen statischen Magnetfeldstärke entspricht, und sehr hohen Feldstärken bzw. Leistungen erforderlich. Zur Verbesserung des Signal-Rauschverhältnisses des von den Antennen empfangenen Magnetresonanzsignals werden häufig als Lokalspulen bezeichnete Antennen verwendet, die unmittelbar am Patienten angeordnet werden.

Zur Bildgebung müssen die von der Lokalspule empfangenen Magnetresonanzsignale zu einer Empfangseinrichtung des Magnetresonanztomographen übertragen werden. Bei einer Lokalspule mit einer Mehrzahl an in einer Matrix angeordneten Antennenspulen kann es sich dabei um eine Mehrzahl an unabhängig voneinander zu übertragenden Signalen handeln.

Zur Übertragung der Signale werden üblicherweise Koaxialkabel verwendet, die insbesondere in dünnen und flexiblen Ausführungsformen teuer und schwierig zu verarbeiten sind. Im Bündel können aber auch dünne Koaxialkabel sperrig und schwer zu handhaben sein.

Aus dem Dokument US 2011/0215807 A1 ist eine Lokalspule zum Einführen in eine Körperöffnung bekannt, die eine Quadraturspule zur Aufnahme von vertikalen und horizontalen Komponenten des Magnetresonanzsignals aufweist. Die elektrische Länge des Anschlusskabels und eines Phasenschieberelements erlauben eine Entkopplung der Lokalspule von einem Anregungs-RF-Feld.

Ein vergleichbarer Gegenstand ist auch aus dem Dokument WO 2012/003211 A1 bekannt, das unter Beteiligung eines gemeinsamen Erfinders entstand.

Es ist daher die Aufgabe der Erfindung, eine Übertragungsvorrichtung und einen Magnetresonanztomographen bereitzustellen, die einfacher zu handhaben und kostengünstiger sind.

Die Aufgabe wird durch eine erfindungsgemäße Übertragungsvorrichtung nach Anspruch 1 sowie einen erfindungsgemäßen Magnetresonanztomographen nach Anspruch 8 und ein Übertragungsverfahren nach Anspruch 12 gelöst.

Die erfindungsgemäße Übertragungsvorrichtung ist zum Übertragen eines ersten Hochfrequenzsignals und eines zweiten Hochfrequenzsignals für einen Magnetresonanztomographen vorgesehen. Das erste Hochfrequenzsignal und das zweite Hochfrequenzsignal unterscheiden sich, insbesondere ist das zweite Hochfrequenzsignal auch nicht ein invertiertes erstes Hochfrequenzsignal. Mit anderen Worten, das erste und das zweite Hochfrequenzsignal sind voneinander unabhängige Signale und werden nicht nur unmittelbar voneinander abgleitet, beispielsweise durch ein aktives oder passives Bauelement. Das erste und das zweite Hochfrequenzsignal unterscheidet sich damit von den sonst üblichen Signalen auf einer symmetrischen Leitung, die sich lediglich durch das Vorzeichen der Amplitude unterscheiden und beispielsweise durch einen Transformator, ein komplexes Netzwerk oder einen Transistor aus einem gemeinsamen Signal erzeugt werden.

Die Übertragungsvorrichtung weist eine geschirmte symmetrische Übertragungsleitung zur Übertragung des ersten Hochfrequenzsignals und des zweiten Hochfrequenzsignals auf. Beispielsweise kann es sich bei der geschirmten symmetrischen Übertragungsleitung um ein geschirmtes Adernpaar eines LAN-Kabels handeln, es sind aber auch andere symmetrische Übertragungsleitungen wie Stegleitung, Streifenleiter auf einer flexiblen Platine oder ähnliches denkbar. Die geschirmte symmetrische Übertragungsleitung der erfindungsgemäßen Übertragungsvorrichtung weist mehrere Adernpaare, darunter ein Paar aus einer ersten Ader und einer zweiten Ader, auf, welche in einem gemeinsamen Schirm geführt werden, ohne gegeneinander durch paarweise Schirmung geschirmt zu sein.

Weiterhin weist die Übertragungsvorrichtung einen ersten Signaltreiber auf, wobei ein erster Signaleingang des ersten Signaltreibers ausgelegt ist, das erste Hochfrequenzsignal zu empfangen und wobei ein erster Signalausgang des ersten Signaltreibers mit der ersten Ader der symmetrischen Übertragungsleitung in elektrischer Verbindung steht. Der erste Signaltreiber ist vorzugsweise eine aktive Verstärkerschaltung, es sind aber auch passive Netzwerke als Signaltreiber denkbar.

Die Übertragungsvorrichtung weist darüber hinaus einen zweiten Signaltreiber auf, wobei ein zweiter Signaleingang des zweiten Signaltreibers ausgelegt ist, das zweite Hochfrequenzsignal zu empfangen und wobei ein zweiter Signalausgang des zweiten Signaltreibers mit der zweiten Ader der symmetrischen Übertragungsleitung in elektrischer Verbindung steht. Im Übrigen lassen sich die für den ersten Signaltreiber gemachten Aussagen auch auf den zweiten Signaltreiber anwenden.

Bei der erfindungsgemäßen Übertragungsvorrichtung steht eine Schirmung der symmetrischen Übertragungsleitung mit einem gemeinsamen Massepotential bzw. Bezugspotential für den ersten Signalausgang und den zweiten Signalausgang in elektrischer Verbindung. Vorzugsweise ist diese Verbindung ohmsch und/oder weist einen geringen Widerstand auf, beispielsweise kleiner als 10 Ohm, 5 Ohm, 1 Ohm oder 0,1 Ohm.

Auf vorteilhafte Weise ermöglicht die erfindungsgemäße Übertragungsvorrichtung die Nutzung einzelner Adern einer geschirmten symmetrischen Übertragungsleitung als einzelne, schwach gekoppelte Koaxialkabel zur im Wesentlichen unabhängigen Übertragung zweier unterschiedlicher Signale. So kann ein kostengünstiges und einfacher handzuhabendes Kabel für einen Magnetresonanztomographen genutzt werden.

Der erfindungsgemäße Magnetresonanztomograph nach Anspruch 8 und das erfindungsgemäße Übertragungsverfahren nach Anspruch 12 teilen die Vorteile der erfindungsgemäßen Übertragungsvorrichtung nach Anspruch 1.

Weitere vorteilhafte Ausführungsformen sind in den abhängigen Ansprüchen angegeben.

In einer möglichen Ausführungsform der erfindungsgemäßen Übertragungsvorrichtung ist die Übertragungsvorrichtung ausgelegt, das erste Hochfrequenzsignal und das zweite Hochfrequenzsignal unabhängig voneinander zu übertragen. Unabhängig voneinander zu übertragen heißt dabei im Sinne der Erfindung, dass am Ende der Übertragungsleitung, das dem Einspeisepunkt der Signaltreiber entgegengesetzt ist, das erste Hochfrequenzsignal an der ersten Ader bereitgestellt wird und das zweite Hochfrequenzsignal an der ersten Ader eine Dämpfung von mehr als 20dB, 30dB oder 40dB gegenüber dem ersten Hochfrequenzsignal aufweist. Weiterhin wird am Ende der Übertragungsleitung das zweite Hochfrequenzsignal an der zweiten Ader bereitgestellt und das erste Hochfrequenzsignal weist an der zweiten Ader eine Dämpfung von mehr als 20dB, 30dB oder 40dB gegenüber dem zweiten Hochfrequenzsignal auf.

In einer denkbaren Ausführungsform der erfindungsgemäßen Übertragungsvorrichtung entspricht eine Länge der Übertragungsleitung im Wesentlichen einem ganzzahligen Vielfachen m einer halben effektiven Wellenlänge des ersten Hochfrequenzsignals und einem ganzzahligen Vielfachen n im Wesentlichen einer halben effektiven Wellenlänge des zweiten Hochfrequenzsignals. Die effektive Wellenlänge wird dabei auf die Mittelfrequenz bezogen und hängt von einer Vakuumwellenlänge durch eine Verkürzungsfaktor ab, der von der Geometrie der geschirmten symmetrischen Übertragungsleitung und verwendeten Materialien abhängt, insbesondere deren Dielektrizitätskonstante. Als im Wesentlichen entsprechen wird dabei angesehen, dass die Länge der Übertragungsleitung um weniger als 5%, 10%, 20 % oder 30% von einem ganzzahligen Vielfachen der halben effektiven Wellenlängen abweicht. Vorzugsweise sind die effektiven Wellenlängen des ersten Hochfrequenzsignals und des zweiten Hochfrequenzsignals im Wesentlichen gleich und damit die ganzen Zahlen n und m gleich. Als im Wesentlichen gleich kann dabei angesehen werden, dass die Frequenzbereiche beider Signale überlappen oder sich die Mittenfrequenzen um höchstens 1%, 5%, 10% oder 20% unterscheiden. Es ist aber auch denkbar, dass das zu übertragendende erste Hochfrequenzsignal und das zweite Hochfrequenzsignal unterschiedliche Mittenfrequenzen aufweisen und m und n verschiedene ganze Zahlen sind.

Bei Längen der Übertragungsleitung, die einem ganzzahligen Vielfachen der halben effektiven Wellenlänge entsprechen, ergibt sich auf vorteilhafte Weise ein Maximum der Entkopplung des ersten und des zweiten Hochfrequenzsignals, sodass eine Dämpfung des jeweils anderen Signals am Ende der Übertragungsleitung von mehr als 20dB, 30dB oder 40dB erreicht werden kann. In einem Magnetresonanztomographen werden darüber hinaus Frequenzraster bei der Auswahl verwendeter Frequenzen genutzt. Um Störungen durch Oberwellen zu vermeiden, werden vorzugsweise Frequenzen mit einem Vielfachen einer Grundfrequenz genutzt. Daher lassen sich auch Längen der

Übertragungsleitung bestimmen, bei denen für unterschiedliche Signalfrequenzen die Bedingung erfüllt ist.

In einer möglichen Ausführungsform der erfindungsgemäßen Übertragungsvorrichtung ist an einem von dem ersten Signaltreiber und dem zweiten Signaltreiber entfernten Ende der Übertragungsleitung die erste Ader der symmetrischen Übertragungsleitung mit der zweiten Ader über einen Entkopplungswiderstand elektrisch verbunden.

Ein Widerstand an einem von dem ersten Signaltreiber und dem zweiten Signaltreiber entfernten Ende, auch als lastseitiges Ende der Übertragungsleitung bezeichnet, ist aufgrund der Phasenverschiebung über die Länge der Übertragungsleitung in der Lage, ein zwischen den Adern auftretendes Übersprechen zumindest teilweise zu kompensieren und so die Übertragungseigenschaften weiter zu verbessern.

In einer denkbaren Ausführungsform der erfindungsgemäßen Übertragungsvorrichtung weist der Entkopplungswiderstand eine komplexe Impedanz auf.

Auf vorteilhafte Weise kann ein komplexer Entkopplungswiderstand zwischen den Adern am lastseitigen Ende durch geeignete Frequenz- und Phasenabhängigkeit ein Übersprechen der Übertragungsleitung noch besser kompensieren.

In einer möglichen Ausführungsform der erfindungsgemäßen Übertragungsvorrichtung weist diese eine Mehrzahl an symmetrischen Übertragungsleitungen, ersten Signaltreibern und zweiten Signaltreiben zur Übertragung einer Mehrzahl an ersten Hochfrequenzsignalen und zweiten Hochfrequenzsignalen auf. Dabei weisen mehrere oder alle symmetrischen Übertragungsleitungen eine gemeinsame Schirmung auf, die diese dann gemeinsam umgibt.

Die erfindungsgemäße Übertragungsvorrichtung ermöglicht durch die Nutzung von Kabeln mit einer Mehrzahl an symmetrischen Adernpaaren, beispielsweise Twisted-Pair-LAN-Kabel, eine kostengünstige Übertragung einer Vielzahl von Hochfrequenzsignalen.

In einer denkbaren Ausführungsform der erfindungsgemäßen Übertragungsvorrichtung sind die erste Ader und die zweite Ader der symmetrischen Übertragungsleitung an einem von dem ersten Signaltreiber und dem zweiten Signaltreiber entfernten Ende der Übertragungsleitung durch jeweils einen Abschlusswiderstand zur Schirmung abgeschlossen. Der Abschlusswiderstand weist dabei vorzugsweise eine Impedanz auf, die der Impedanz der Übertragungsleitung im Wesentlichen entspricht, der Betrag des Abschlusswiderstandes also höchstens um 10%, 20%, 50% oder 100% von einem Betrag der Impedanz der Übertragungsleitung abweicht. Aufgrund der nachfolgend in der Figurenbeschreibung beschriebenen Lambda/2-Transformation sind aber auch andere Werte für die Impedanz des Abschlusswiderstands denkbar.

Auf vorteilhafte Weise werden durch einen Abschluss der Übertragungsleitung mit einem Widerstand, der der Impedanz der Übertragungsleitung entspricht, Reflektionen auf der Übertragungsleitung reduziert.

In einer möglichen Ausführungsform der erfindungsgemäßen Übertragungsvorrichtung weist das erste Hochfrequenzsignal eine erste Mittenfrequenz und das zweite Hochfrequenzsignal eine zweite Mittenfrequenz aufweist, wobei die erste Mittenfrequenz und die zweite Mittenfrequenz unterschiedlich sind. Als unterschiedlich wird dabei angesehen, dass die erste Mittenfrequenz von der zweiten Mittenfrequenz um mehr als 30%, 50%, 100% oder um ein mehrfaches abweicht.

Auf vorteilhafte Weise ist die erfindungsgemäße Übertragungsvorrichtung in der Lage, unterschiedliche Signale auch in unterschiedlichen Frequenzbereichen unabhängig voneinander zu übertragen, wobei als unabhängig eine Entkopplung des ersten Hochfrequenz von dem zweiten Hochfrequenzsignal an einem von den Signaltreibern entgegengesetzten Ende der Übertragungsleitung eine Dämpfung des jeweils anderen Signals von mehr als 10 dB, 20 dB, 30 dB oder 40 dB angesehen wird.

Das erfindungsgemäße Verfahren teilt die Vorteile der erfindungsgemäßen Vorrichtung.

Die oben beschriebenen Eigenschaften, Merkmale und Vorteile dieser Erfindung sowie die Art und Weise, wie diese erreicht werden, werden klarer und deutlicher verständlich im Zusammenhang mit der folgenden Beschreibung der Ausführungsbeispiele, die im Zusammenhang mit den Zeichnungen näher erläutert werden.

Es zeigen:
- Fig. 1: eine beispielhafte schematische Darstellung einer Ausführungsform eines erfindungsgemäßen Magnetresonanztomographen;
- Fig. 2: eine beispielhafte schematische Darstellung einer Ausführungsform einer erfindungsgemäßen Übertragungsvorrichtung;
- Fig. 3: eine beispielhafte schematische Darstellung einer Ausführungsform einer erfindungsgemäßen Übertragungsvorrichtung;
- Fig. 4: einen beispielhaften Verlauf einer Übersprechungsdämpfung in einer erfindungsgemäßen Übertragungseinrichtung.

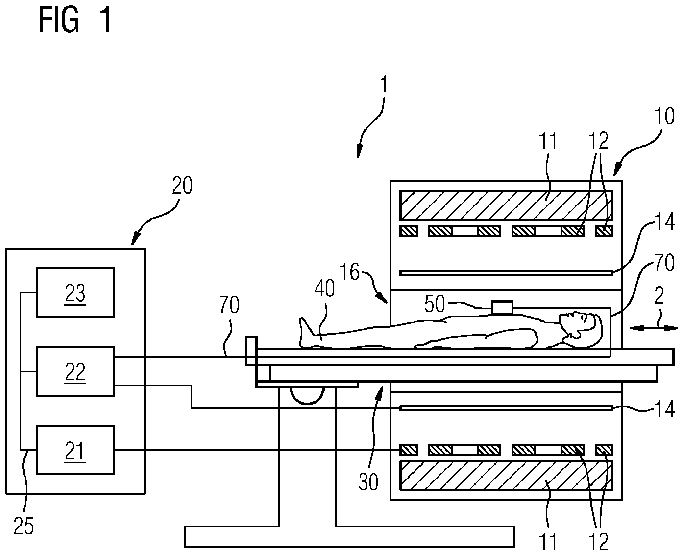

Fig. 1 zeigt eine schematische Darstellung einer Ausführungsform eines erfindungsgemäßen Magnetresonanztomographen 1 mit einer erfindungsgemäßen Übertragungsvorrichtung 70.

Die Magneteinheit 10 weist einen Feldmagneten 11 auf, der ein statisches Magnetfeld B0 zur Ausrichtung von Kernspins von Proben bzw. in einem Körper eines Patienten 40 in einem Aufnahmebereich erzeugt. Der Aufnahmebereich ist in einem Patiententunnel 16 angeordnet, der sich in einer Längsrichtung 2 durch die Magneteinheit 10 erstreckt. Üblicherweise handelt es sich bei dem Feldmagneten 11 um einen supraleitenden Magneten, der magnetische Felder mit einer magnetischen Flussdichte von bis zu 3T, bei neuesten Geräten sogar darüber, bereitstellen kann. Für geringere Feldstärken können jedoch auch Permanentmagnete oder Elektromagnete mit normalleitenden Spulen Verwendung finden.

Weiterhin weist die Magneteinheit 10 Gradientenspulen 12 auf, die dazu ausgelegt sind, zur räumlichen Differenzierung der erfassten Abbildungsbereiche in dem Untersuchungsvolumen dem Magnetfeld B0 variable Magnetfelder in drei Raumrichtungen zu überlagern. Die Gradientenspulen 12 sind üblicherweise Spulen aus normalleitenden Drähten, die zueinander orthogonale Felder in dem Untersuchungsvolumen erzeugen können.

Die Magneteinheit 10 weist ebenfalls eine Körperspule 14 auf, die dazu ausgelegt ist, ein über eine Signalleitung zugeführtes Hochfrequenzsignal in das Untersuchungsvolumen abzustrahlen und von dem Patient 40 emittierte Resonanzsignale zu empfangen und über eine Signalleitung abzugeben. Weiterhin weist der erfindungsgemäße Magnetresonanztomograph eine oder mehrere Lokalspulen 50 auf, die in dem Patiententunnel 16 nahe am Patient 40 angeordnet sind.

Eine Steuereinheit 20 versorgt die Magneteinheit 10 mit den verschiedenen Signalen für die Gradientenspulen 12 und die Körperspule 14 und wertet die empfangenen Signale aus.

So weist die Steuereinheit 20 eine Gradientenansteuerung 21 auf, die dazu ausgelegt ist, die Gradientenspulen 12 über Zuleitungen mit variablen Strömen zu versorgen, welche zeitlich koordiniert die erwünschten Gradientenfelder in dem Untersuchungsvolumen bereitstellen.

Weiterhin weist die Steuereinheit 20 eine Empfangseinheit 22 auf, die ausgelegt ist, einen Hochfrequenz-Puls mit einem vorgegebenen zeitlichen Verlauf, Amplitude und spektraler Leistungsverteilung zur Anregung einer Magnetresonanz der Kernspins in dem Patienten 40 zu erzeugen. Dabei können Pulsleistungen im Bereich von Kilowatt erreicht werden. Die einzelnen Einheiten sind über einen Signalbus 25 untereinander verbunden.

Die Lokalspule 50 empfängt vorzugsweise ein Magnetresonanzsignal aus dem Körper des Patienten 40, denn aufgrund des geringen Abstandes ist das Signal-zu-Rauschverhältnis (SNR) der Lokalspule 50 besser als bei einem Empfang durch die Körperspule 14. Das von der Lokalspule 50 empfangene MR-Signal wird in der Lokalspule 50 aufbereitet und mittels der erfindungsgemäßen Übertragungsvorrichtung 70 an die Empfangseinheit 22 des Magnetresonanztomographen 1 zur Auswertung und Bilderfassung weitergeleitet. Dabei können Teile der Übertragungsvorrichtung 70 auch in einem Gehäuse der Lokalspule 50 vorgesehen sein.

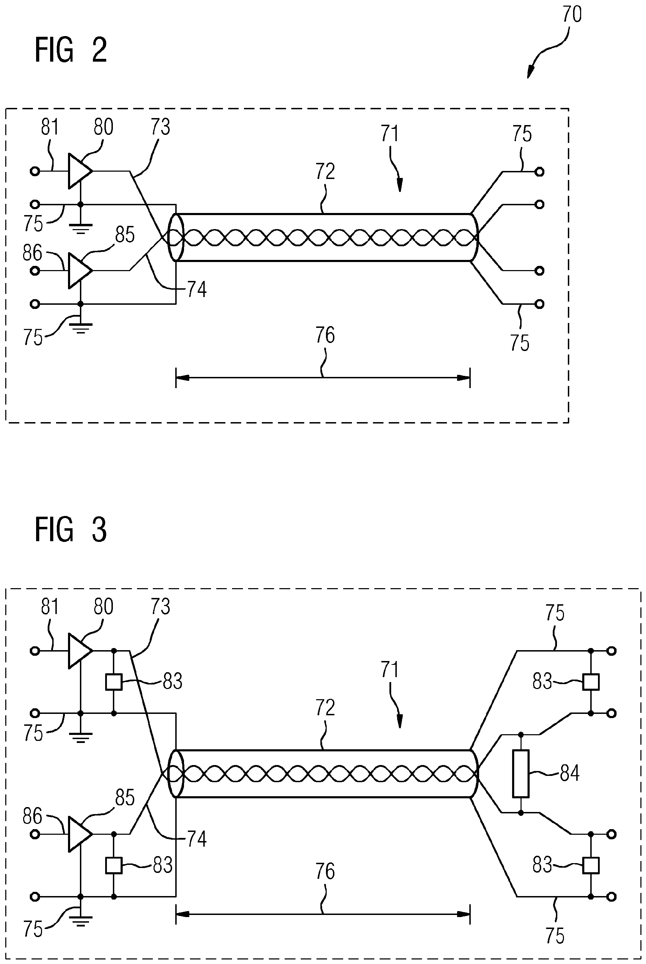

Fig. 2 zeigt eine mögliche Ausführungsform einer erfindungsgemäßen Übertragungsvorrichtung in schematischer Darstellung. Die erfindungsgemäße Übertragungsvorrichtung 70 weist eine symmetrische Übertragungsleitung 71 auf, bei der sich eine erste Ader 73 und eine zweite Ader 74 durch ein Dielektrikum getrennt, von einer Schirmung 72 umgeben, entlang einer Längsausdehnung der Übertragungsleitung 71 erstrecken. Die erste Ader 73 und die zweite Ader 74 können dabei wie in der Zeichnung angedeutet miteinander verdrillt sein. Beispiele für derartige Übertragungsleitungen sind LAN-Kabel bei denen mehrere Adernpaare gebündelt sind. Die Schirmung 72 kann dabei Beispielsweise durch eine leitende Folie oder von einem Drahtgeflecht gebildet sein. Es ist aber auch denkbar, dass die erste Ader 73 und die zweite Ader 74 in einem im Wesentlichen vorbestimmten Abstand parallel zueinander entlang der Längsausdehnung angeordnet sind.

In einer Ausführungsform können die erste Ader 73 und die zweite Ader 74 sowie die Schirmung 72 an einem oder beiden Enden der Übertragungsleitung mit einem Steckverbinder , beispielsweise einem RJ-45 Stecker, für eine lösbare elektrische Verbindung konfektioniert sein.

In der symmetrischen Übertragungsleitung 71 der erfindungsgemäßen Übertragungsvorrichtung 70 werden mehrere Adernpaare in einem gemeinsamen Schirm geführt, ohne gegeneinander durch paarweise Schirmung geschirmt zu sein, beispielsweise LAN CAT-6 Kabel mit der Bezeichnung S/UTP ("Screened Unshielded Twisted Pair") .

Die Übertragungsvorrichtung 70 weist weiterhin einen ersten Signaltreiber 80 mit einem ersten Signaleingang 81 und einen zweiten Signaltreiber 85 mit einem zweiten Signaleingang 86 zur Zuführung von zwei unabhängigen Hochfrequenzsignalen auf, jeweils eines der Hochfrequenzsignale für einen Signaltreiber. Als Bezugspotential für das erste und das zweite Hochfrequenzsignal dient ein Bezugspotential bzw. Massepotential 75. Als unabhängig werden dabei die Hochfrequenzsignale angesehen, wenn diese nicht unmittelbar voneinander durch eine einfache passive oder aktive Schaltung abgeleitet werden. Insbesondere werden nicht solche Hochfrequenzsignale als unabhängig betrachtet, die üblicherweise für die Verwendung von symmetrischen Übertragungsleitungen aus einem einzigen Signal durch Invertierung bzw. Phasenverschiebung erzeugt werden, sodass die erste Ader und die zweite Ader jeweils entgegengesetzte Spannung führen und ein übersprechendes Signal durch das entgegengesetzte Signal der anderen Ader kompensiert wird. Auch ist das Summensignal unabhängiger Hochfrequenzsignale nicht dauerhaft im Wesentlichen gleich Null, sodass für das erste Hochfrequenzsignal und das zweite Hochfrequenzsignal nach der Übertragungsleitung ein Bezugspotential bzw. eine Signalmasse bereitgestellt werden muss.

Das erste und das zweite Hochfrequenzsignal wird in dem ersten Signaltreiber 80 und dem zweiten Signaltreiber 85 verstärkt und/oder an eine Impedanz angepasst, die an dem ersten Signalausgang 82 bzw. dem zweiten Signalausgang 87 angeschlossen ist. Dabei ist es sowohl denkbar, dass der erste Signaltreiber 80 und der zweite Signaltreiber 85 aktive Verstärkerschaltungen sind oder auch nur passive Anpassungsnetzwerke.

Der erste Signalausgang 82 ist dabei mit der ersten Ader 73 elektrisch verbunden und der zweite Signalausgang 87 mit der zweiten Ader 74. Eine Impedanz der ersten Ader 73 und der zweiten Ader 74 als Lastimpedanz an den Signalausgängen ist dabei durch die Geometrie der Übertragungsleitung 71 sowie der verwendeten Materialien bestimmt. Auch kann wie nachfolgend zu Fig. 3 erläutert, die Impedanz durch Abschlusswiderstände beeinflusst sein. Weiterhin ist das Massepotential 75, das das Bezugspotential für den ersten Signaleingang 81 und den zweiten Signaleingang 86 bildet, auch gleichzeitig das Bezugspotential für den ersten Signalausgang 82 und den zweiten Signalausgang 87 und sowohl mit dem ersten Signaltreiber 80, dem zweiten Signaltreiber 85 und der Schirmung 72 elektrisch verbunden, vorzugsweise ohmsch.

Der erste Signaltreiber 80 und der zweite Signaltreiber 85 sind vorzugsweise in einem Gehäuse der Lokalspule 50 vorgesehen, es ist aber genauso denkbar, dass der erste Signaltreiber 80 und der zweite Signaltreiber 85 in einem separaten Gehäuse, in einem Gehäuse eines Steckverbinders oder bei einer Signalübertragung von der Steuereinheit 20 hin zu der Lokalspule 50 auch in einem Gehäuse der Steuereinheit 20 als ein Bestandteil vorgesehen ist.

Vorzugsweise weist die Übertragungsleitung 71 eine Länge 76 auf, die ein ganzzahliges Vielfaches der verkürzten effektiven halben Wellenlänge des ersten und des zweiten Hochfrequenzsignals ist. Ein Verkürzungsfaktor ergibt sich durch die Geometrie und die verwendeten Materialien, insbesondere deren Dielektrizitätskonstante, der Übertragungsleitung 71. Ein typischer Wert für den Verkürzungsfaktor ist bei einem CAT-7 Kabel zwischen 0,6 und 0,7. Für Längen 76, die ein Vielfaches der verkürzten halben Wellenlänge des ersten und/oder zweiten Hochfrequenzsignals sind, ergeben sich Minima im Übersprechen zwischen dem ersten und dem zweiten Hochfrequenzsignal an dem Ende der Übertragungsleitung 71, das dem ersten Signaltreiber 80 und dem zweiten Signaltreiber 85 entgegengesetzt ist.

Zudem reduziert sich bei diesen Längenverhältnissen der Einfluss der vorliegenden Leitungsimpedanz auf die Signalübertragung, da aufgrund der Lambda/2-Transformation am Leitungs-Eingang stets die am Leitungs-Ende vorliegende Lastimpedanz erscheint. Hierdurch wird es möglich, Kabelanordnungen zu verwenden, die nicht für diesen Anwendungsfall auf zum Beispiel 50 Ω Leitungsimpedanz ausgelegt wurden.

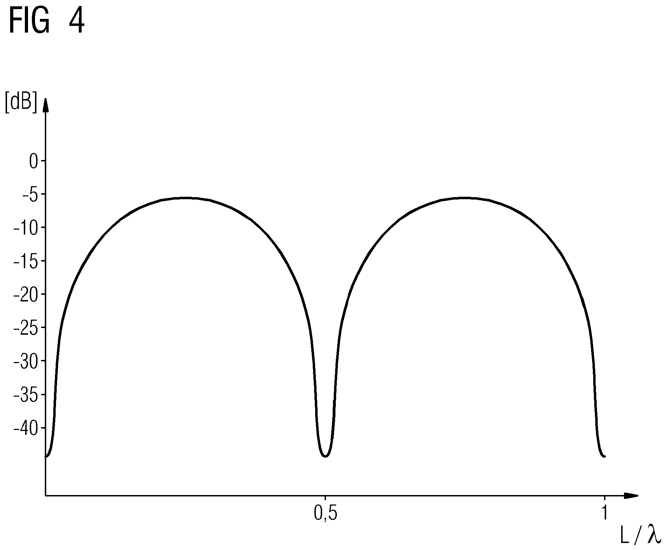

In Fig. 4 ist ein beispielhafter Verlauf der Übersprechungsdämpfung in einer erfindungsgemäßen Übertragungseinrichtung dargestellt. Die horizontale Achse gibt das Verhältnis der Länge durch die effektive Wellenlänge an, das entspricht gleichzeitig einer zunehmenden Frequenz nach rechts. Die Hochachse gibt die den Pegel des jeweils anderen Hochfrequenzsignals in Dezibel in Bezug auf das zu übertragende Hochfrequenzsignal an dem Ende der Übertragungsleitung 71 an, das dem ersten Signaltreiber 80 und dem zweiten Signaltreiber 85 entgegengesetzt ist. Dabei schwankt der Pegel zwischen -5 dB bei ungünstiger Länge 76 der Übertragungsleitung 71 und - 40 dB bei einer idealen Länge L = Lambda_{eff}^{∗}n/2 mit n als natürlicher Zahl. Die maximale Dämpfung ist dabei abhängig von den Eigenschaften der Übertragungsleitung und kann typische Werte zwischen 15 dB und 45 dB oder mehr annehmen. Die Dämpfung ergibt sich als der Betrag des Pegelverhältnisses. Weisen das erste und das zweite Hochfrequenzsignal unterschiedliche Mittenfrequenzen und effektive Wellenlängen λ₁, λ₂ auf, so ist es in einer Ausführungsform der erfindungsgemäßen Übertragungsvorrichtung 70 möglich, die Länge der Übertragungsleitung 70 L als L = (n^{∗}λ₁)/2 = (m^{*}λ₂)/2 mit n, m als natürliche Zahlen zu wählen.

Aus der Fig. 4 ist auch ersichtlich, dass das Minimum der Übersprechungsdämpfung eine gewisse Breite in Bezug auf Länge zur effektiven Wellenlänge und damit auch in Bezug auf die Frequenz des ersten bzw. zweiten Hochfrequenzsignals aufweist. Auf vorteilhafte Weise ist es daher auch möglich, ein Signal mit einer gewissen Bandbreite zu übertragen, ohne dass die Übersprechungsdämpfung zu groß wird. Hier ist zwischen geforderter Bandbreite, minimal zulässiger Übersprechungsdämpfung in Abhängigkeit von den Eigenschaften der Übertragungsleitung 71 abzuwägen.

Fig. 3 stellt eine denkbare Ausführungsform der erfindungsgemäßen Übertragungsvorrichtung 70 dar. Gleiche Gegenstände sind mit gleichen Referenzzeichen bezeichnet.

Die Übertragungsvorrichtung 70 der Fig. 3 unterscheidet sich zum einen von der Übertragungsvorrichtung der Fig. 2 durch die Abschlusswiderstände 83. Mittels der Abschlusswiderstände 83 können Reflexionen auf der Übertragungsleitung 71 reduziert werden, wenn die Ausgangsimpedanz des ersten Signaltreibers 80 und des zweiten Signaltreibers 85 nicht der Leitungsimpedanz der Übertragungsleitung 71 entsprechen oder eine an dem den Signaltreibern entgegengesetzte Ende angeschlossene Last eine andere Eingangsimpedanz aufweist.

Gleichzeitig oder auch unabhängig von den Abschlusswiderständen 83 ist es denkbar, die erste Ader 73 und die zweite Ader 74 an dem den Signaltreibern entgegengesetzte Ende der Übertragungsleitung 71 durch einen Entkopplungswiderstand 84 zu verbinden. Aufgrund der Phasenverschiebung durch die Länge 76 der Übertragungsleitung 71 mit L = Lambda_{eff}^{∗}n/2 kann durch ein entgegengesetztes Vorzeichen des ersten Hochfrequenzsignals zu dem auf die zweite Ader 74 eingekoppelte Übersprechungssignal die Übersprechungsdämpfung verbessert werden, und umgekehrt für das zweite Hochfrequenzsignal.

In den Fig. 2 und Fig. 3 ist die elektrische Verbindung zwischen dem ersten Signalausgang 82 und der ersten Ader 73 bzw. dem zweiten Signalausgang 87 und der zweiten Ader 74 nur schematisch angedeutet und die Art der elektrischen Verbindung nicht genauer festgelegt. Bei dieser elektrischen Verbindung kann es sich bei einer geringen Länge, beispielsweise einer Länge kleiner als ein Zehntel der effektiven Wellenlänge Lambda_{eff} des zu übertragenden Hochfrequenzsignals um einfache elektrische Drahtverbindungen oder Leitungen auf einer Platine. Bei größeren Längen werden jedoch Wellenphänomene wie Reflektion relevant, sodass vorzugsweise Wellenleiter wie zum Beispiel Koaxialleitungen oder Streifenleiter, gegebenen Falls mit Abschlusswiderständen, die elektrische Verbindung bereitstellen.

Die Übertragungsleitung 71 der erfindungsgemäßen Übertragungsvorrichtung 70 weist eine Mehrzahl an Paaren von erster Ader 73 und zweiter Ader 74 auf, die zusammengefasst lediglich mit einer gemeinsamen Schirmung 72 versehen sind, die jedoch nicht paarweise mit einer separaten Schirmung 72 versehen sind.

Hierunter fallen beispielsweise LAN-Kabel mit der Bezeichnung S/UTP ("Screened Unshielded Twisted Pair").

Das erste Hochfrequenzsignal oder das zweite Hochfrequenzsignal können beispielsweise Magnetresonanzsignale sein, die von mehreren Antennenspulen einer Lokalspule 50 aufgenommen werden. Diese Signale können dabei beispielsweise durch einen Vorverstärker (LNA) verstärkt sein, oder auch durch Mischung mit einem lokalen Oszillator auf eine niedrigere Zwischenfrequenz umgesetzt sein. Dabei ist es denkbar, dass zur weiteren Verarbeitung das lokale Oszillatorsignal als Referenz benötigt wird und ebenfalls eines der zu übertragenden Hochfrequenzsignale ist.

In einer denkbaren Ausführungsform des erfindungsgemäßen Magnetresonanztomographen 1 kann eine weitere Kompensation des Übersprechens in einer der Übertragungsvorrichtung nachfolgenden Signalverarbeitung realisiert werden. Beispielsweise kann die Empfangseinheit 22 durch ein passives Kompensationsnetzwerk oder auch durch eine digitale Signalbearbeitung ausgelegt sein, Anteile des zweiten Hochfrequenzsignals in dem übertragenen ersten Hochfrequenzsignal zu reduzieren. Wenn die Eigenschaften der Übertragungsvorrichtung 70 bekannt sind, insbesondere der Grad des Übersprechens und die Phasenbeziehung, so kann digital oder analog ein entsprechendes Kompensationssignal mit umgekehrtem Vorzeichen erzeugt werden und durch Summation der Signale eine weitere Reduzierung des Übersprechens erreicht werden.

Das erfindungsgemäße Verfahren zum Übertragen eines ersten Hochfrequenzsignals und eines zweiten Hochfrequenzsignals wird auf einem erfindungsgemäßen Magnetresonanztomographen 1 mit einer bereits beschriebenen erfindungsgemäßen Übertragungsvorrichtung 70 ausgeführt. Bei dem Übertragungsverfahren wird das erste Hochfrequenzsignal dem ersten Signaleingang 81 des ersten Signaltreibers 80 zugeführt wird und an dem ersten Signalausgang 82 an die erste Ader 73 der Übertragungsleitung 71 weitergeleitet. Ebenso wird das zweite Hochfrequenzsignal dem zweiten Signaleingang 86 des zweiten Signaltreibers 85 zugeführt und an dem zweiten Signalausgang 87 an die zweite Ader 74 weitergeleitet. Das erste Hochfrequenzsignal und das zweite Hochfrequenzsignal sind voneinander unabhängige Signale in dem bereits beschriebenen Sinn, insbesondere lässt sich das zweite Hochfrequenzsignal nicht durch eine einfache passive oder aktive Schaltung aus dem ersten Hochfrequenzsignal ableiten, und umgekehrt. Das erste Hochfrequenzsignal und das zweite Hochfrequenzsignal können beispielsweise Magnetresonanzsignale unterschiedlicher Antennenspulen aus unterschiedlichen Körperbereichen sein. Das erste Hochfrequenzssignal und das zweite Hochfrequenzsignal werden erfindungsgemäß zu einem von dem ersten Signaltreiber und dem zweiten Signaltreiber entfernten Ende der Übertragungsleitung unabhängig voneinander übertragen, mit anderen Worten, das erste Hochfrequenzsignal und das zweite Hochfrequenzsignal sind nach der Übertragung im Wesentlichen unverändert, d.h. bis auf eine konstante Dämpfung und Phasenverschiebung. Insbesondere ist eine Übersprechungsdämpfung zwischen den beiden Hochfrequenzsignalen größer als 15, 20, 30 oder 40 dB.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen, sofern sie unter den Gegenstand der Ansprüche fallen.

## Patentansprüche

1. Übertragungsvorrichtung zum Übertragen eines ersten Hochfrequenzsignals und eines zweiten Hochfrequenzsignals für einen Magnetresonanztomographen (1), wobei die Übertragungsvorrichtung (70) aufweist:
eine geschirmte symmetrische Übertragungsleitung (71) zur Übertragung des ersten Hochfrequenzsignals und des zweiten Hochfrequenzsignals,
einen ersten Signaltreiber (80), wobei ein erster Signaleingang (81) des ersten Signaltreibers (80) ausgelegt ist, das erste Hochfrequenzsignal zu empfangen und wobei ein erster Signalausgang (82) des ersten Signaltreibers (80) mit einer ersten Ader (73) der symmetrischen Übertragungsleitung (71) in elektrischer Verbindung steht;
einen zweiten Signaltreiber (85), wobei ein zweiter Signaleingang (86) des zweiten Signaltreibers (85) ausgelegt ist, das zweite Hochfrequenzsignal zu empfangen und wobei ein zweiter Signalausgang (87) des zweiten Signaltreibers (85) mit einer zweiten Ader (74) der symmetrischen Übertragungsleitung (71) in elektrischer Verbindung steht und wobei eine Schirmung (72) der symmetrischen Übertragungsleitung (71) mit einem gemeinsamen Massepotential (75) für den ersten Signalausgang (82) und den zweiten Signalausgang (87) in elektrischer Verbindung steht,
**dadurch gekennzeichnet, dass** in der Übertragungsleitung (71) mehrere Adernpaare, darunter das Paar aus erster Ader (73) und zweiter Ader (74), in einem gemeinsamen Schirm geführt werden, ohne gegeneinander durch paarweise Schirmung geschirmt zu sein.

2. Übertragungsvorrichtung nach Anspruch 1, wobei die Übertragungsvorrichtung (70) ausgelegt ist, das erste Hochfrequenzsignal und das zweite Hochfrequenzsignal unabhängig voneinander zu übertragen.

3. Übertragungsvorrichtung nach Anspruch 1 oder 2, wobei eine Länge (76) der Übertragungsleitung (71) im Wesentlichen einem ganzzahligen Vielfachen m einer halben effektiven Wellenlänge des ersten Hochfrequenzsignals entspricht und im Wesentlichen einem ganzzahligen Vielfachen n einer halben effektiven Wellenlänge des zweiten Hochfrequenzsignals.

4. Übertragungsvorrichtung nach einem der vorhergehenden Ansprüche, wobei an einem von dem ersten Signaltreiber (80) und dem zweiten Signaltreiber (85) entfernten Ende der Übertragungsleitung (71) die erste Ader (73) der symmetrischen Übertragungsleitung (71) mit der zweiten (74) Ader über einen Entkopplungswiderstand (84) elektrisch verbunden ist.

5. Übertragungsvorrichtung nach Anspruch 4, wobei der Entkopplungswiderstand (84) eine komplexe Impedanz aufweist.

6. Übertragungsvorrichtung nach einem der vorhergehenden Ansprüche, wobei die Übertragungsvorrichtung (70) eine Mehrzahl an symmetrischen Übertragungsleitungen (71), ersten Signaltreibern (80) und zweiten Signaltreiben (85) zur Übertragung einer Mehrzahl an ersten Hochfrequenzsignalen und zweiten Hochfrequenzsignalen aufweist.

7. Übertragungsvorrichtung nach einem der vorhergehenden Ansprüche, wobei die erste Ader (73) und die zweite Ader (74) der symmetrischen Übertragungsleitung (71) an einem von dem ersten Signaltreiber (80) und dem zweiten Signaltreiber (85) entfernten Ende der Übertragungsleitung (71) durch jeweils einen, insbesondere einer Leitungsimpedanz der ersten Ader (73) und der zweiten Ader (74) entsprechenden Abschlusswiderstand (83) zur Schirmung (72) abgeschlossen sind.

8. Magnetresonanztomograph mit einer Übertragungsvorrichtung (71) nach einem der vorhergehenden Ansprüche.

9. Magnetresonanztomograph nach Anspruch 8, wobei der Magnetresonanztomograph (1) eine Lokalspule (50) und eine Empfangseinrichtung (22) aufweist und die Übertragungseinrichtung (70) eine Signalverbindung zwischen der Lokalspule (50) und der Empfangseinrichtung (22) herstellt.

10. Magnetresonanztomograph nach Anspruch 8 oder 9, wobei das erste Hochfrequenzsignal und/oder das zweite Hochfrequenzsignal eines aus Magnetresonanzsignal, Zwischenfrequenzsignal oder lokalem Oszillatorsignal ist oder sich aus diesen zu einem kombinierten Signal zusammensetzt und das erste Hochfrequenzsignal eine erste Mittenfrequenz und das zweite Hochfrequenzsignal eine zweite Mittenfrequenz aufweist, wobei die erste Mittenfrequenz und die zweite Mittenfrequenz unterschiedlich sind.

11. Magnetresonanztomograph nach Anspruch 9, oder nach Anspruch 10 sofern von Anspruch 9 abhängig, wobei die Empfangseinrichtung eine digitale Signalverarbeitung zur Verarbeitung der von der Übertragungsvorrichtung empfangenen Signale aufweist und die digitale Signalverarbeitung ausgelegt ist, ein Übersprechen der zwischen den Signalen zumindest teilweise zu kompensieren.

12. Verfahren zum Übertragen eines ersten Hochfrequenzsignals und eines zweiten Hochfrequenzsignals für einen Magnetresonanztomographen (1), mit einer Übertragungsvorrichtung (70) nach Anspruch 1, wobei in einem Schritt des Verfahrens das erste Hochfrequenzsignal dem ersten Signaleingang (81) des ersten Signaltreibers (80) zugeführt wird und an dem ersten Signalausgang (82) an die erste Ader (73) weitergeleitet wird, das zweite Hochfrequenzsignal dem zweiten Signaleingang (86) des zweiten Signaltreibers (85) zugeführt wird und an dem zweiten Signalausgang (87) an die zweite Ader (74) weitergeleitet wird, wobei das erste Hochfrequenzsignal unabhängig von dem zweiten Hochfrequenzsignal ist und das erste Hochfrequenzssignal und das zweite Hochfrequenzsignal zu einem von dem ersten Signaltreiber (80) und dem zweiten Signaltreiber (85) entfernten Ende der Übertragungsleitung (71) unabhängig voneinander übertragen werden.

## Claims

1. Transmitting device for transmitting a first high frequency signal and a second high frequency signal for a magnetic resonance tomograph (1), wherein the transmitting device (70) has:
a shielded balanced transmission line (71) for transmitting the first high frequency signal and the second high frequency signal,
a first signal driver (80), wherein a first signal input (81) of the first signal driver (80) is designed to receive the first high frequency signal, and wherein a first signal output (82) of the first signal driver (80) has an electrical connection to a first conductor (73) of the balanced transmission line (71);
a second signal driver (85), wherein a second signal input (86) of the second signal driver (85) is designed to receive the second high frequency signal, and wherein a second signal output (87) of the second signal driver (85) has an electrical connection to a second conductor (74) of the balanced transmission line (71), and wherein a shielding (72) of the balanced transmission line (71) has an electrical connection to a common ground potential (75) for the first signal output (82) and the second signal output (87),
**characterised in that** a plurality of conductor pairs, including the pair of the first conductor (73) and second conductor (74), are routed in the transmission line (71) in a common shielding without being shielded with respect to each other by means of pair-based shielding.

2. Transmitting device according to claim 1, wherein the transmitting device (70) is designed to transmit the first high frequency signal and the second high frequency signal independently of each other.

3. Transmitting device according to claim 1 or 2, wherein a length (76) of the transmission line (71) essentially corresponds to an integer multiple m of a half effective wavelength of the first high frequency signal and essentially to an integer multiple n of a half effective wavelength of the second high frequency signal.

4. Transmitting device according to one of the preceding claims, wherein the first conductor (73) of the balanced transmission line (71) has an electrical connection to the second (74) conductor by way of a decoupling resistor (84) at an end of the transmission line (71) that is remote from the first signal driver (80) and the second signal driver (85).

5. Transmitting device according to claim 4, wherein the decoupling resistor (84) has a complex impedance.

6. Transmitting device according to one of the preceding claims, wherein the transmitting device (70) has a plurality of balanced transmission lines (71), first signal drivers (80), and second signal drivers (85) for transmitting a plurality of first high frequency signals and second high frequency signals.

7. Transmitting device according to one of the preceding claims, wherein the first conductor (73) and the second conductor (74) of the balanced transmission line (71) are in each case terminated for the purpose of shielding (72) by means of a terminating resistor (83) corresponding in particular to a line impedance of the first conductor (73) and the second conductor (74) at an end of the transmission line (71) that is remote from the first signal driver (80) and the second signal driver (85).

8. Magnetic resonance tomograph with a transmitting device (71) according to one of the preceding claims.

9. Magnetic resonance tomograph according to claim 8, wherein the magnetic resonance tomograph (1) has a local coil (50) and a receiving facility (22), and the transmitting facility (70) creates a signal link between the local coil (50) and the receiving facility (22).

10. Magnetic resonance tomograph according to claim 8 or 9, wherein the first high frequency signal and/or the second high frequency signal is one out of magnetic resonance signal, intermediate frequency signal or local oscillator signal or is assembled from same in to a combined signal and the first high frequency signal has a first centre frequency and the second high frequency signal a second centre frequency, wherein the first centre frequency and the second centre frequency are different.

11. Magnetic resonance tomograph according to claim 9, or according to claim 10, provided this is dependent upon claim 9, wherein the receiving facility has a digital signal processing function for processing the signals received from the transmitting device, and the digital signal processing is designed to compensate at least partly for a crosstalk between the signals.

12. Method for transmitting a first high frequency signal and a second high frequency signal for a magnetic resonance tomograph (1) with a transmitting device (70) according to claim 1,
wherein, in a step of the method, the first high frequency signal is fed to the first signal input (81) of the first signal driver (80) and sent on at the first signal output (82) to the first conductor (73), and the second high frequency signal is fed to the second signal input (86) of the second signal driver (85) and sent on at the second signal output (87) to the second conductor (74),
wherein the first high frequency signal is independent of the second high frequency signal and the first high frequency signal and the second high frequency signal are transmitted independently of each other to an end of the transmission line (71) that is remote from the first signal driver (80) and the second signal driver (85).

## Revendications

1. Système de transmission d'un premier signal de haute fréquence et d'un deuxième signal de haute fréquence pour un tomodensitographe (1) à résonnance magnétique, dans lequel le système (70) de transmission a :
une ligne (71) de transmission symétrique et blindée pour la transmission du premier signal de haute fréquence et du deuxième signal de haute fréquence,
un premier étage (80) d'attaque de signal, une première sortie (81) de signal du premier étage (80) d'attaque du signal étant conçue pour recevoir le premier signal de haute fréquence et une première sortie (82) du signal du premier étage (80) d'attaque de signal étant en liaison électrique avec un premier brin (73) de la ligne (71) de transmission symétrique ;
un deuxième étage d'attaque (85) de signal, une deuxième entrée (86) du signal du deuxième étage d'attaque (85) de signal étant conçue pour recevoir le deuxième signal de haute fréquence et une deuxième sortie (87) du signal du deuxième étage d'attaque (85) de signal étant en liaison électrique avec un deuxième brin (74) de la ligne (71) de transmission symétrique, et dans lequel un blindage (72) de la ligne (71) est en liaison électrique avec un potentiel (75) de masse commun à la première sortie (82) du signal et à la deuxième sortie (87) du signal,
**caractérisé en ce que**, dans la ligne (71) de transmission, passe, dans un blindage commun, plusieurs paires de brins, parmi elles la paire composée du premier brin (73) et du deuxième brin (74), sans être blindées les unes par rapport aux autres par un blindage paire par paire.

2. Système de transmission suivant la revendication 1, dans lequel le système (70) de transmission est conçu pour transmettre le premier signal de haute fréquence et le deuxième signal de haute fréquence indépendamment l'un de l'autre.

3. Système de transmission suivant la revendication 1 ou 2, dans lequel une longueur (76) de la ligne (71) de transmission correspond sensiblement à un multiple m en nombre entier d'une demi longueur d'onde effective du premier signal de haute fréquence et sensiblement à un multiple n en nombre entier d'une demi longueur d'onde effective du deuxième signal de haute fréquence.

4. Système de transmission suivant l'une des revendications précédentes, dans lequel, à un bout, éloigné du premier étage d'attaque (80) de signal et du deuxième étage d'attaque (85) de signal, de la ligne (71) de transmission, le premier brin (73) de la ligne (71) de transmission symétrique est relié électriquement au deuxième brin (74) par l'intermédiaire d'une résistance (84) de découplage.

5. Système de transmission suivant la revendication 4, dans lequel la résistance (84) de découplage a une impédance complexe.

6. Système de transmission suivant l'une des revendications précédentes, dans lequel le système (70) de transmission a une pluralité de lignes (71) de transmission symétriques, de premiers étages d'attaque (80) de signal et de deuxièmes étages d'attaque (85) de signal pour la transmission d'une pluralité de premiers signaux de haute fréquence et de deuxièmes signaux de haute fréquence.

7. Système de transmission suivant l'une des revendications précédentes, dans lequel le premier brin (73) et le deuxième brin (74) de la ligne (71) de transmission symétrique sont séparés du blindage (72) par, respectivement, une résistance (83) de terminaison correspondant notamment à une impédance de ligne du premier brin (73) et du deuxième brin (74).

8. Tomodensitographe à résonance magnétique ayant un système (71) de transmission suivant l'une des revendications précédentes.

9. Tomodensitographe à résonance magnétique suivant la revendication 8, dans lequel le tomodensitographe (1) à résonance magnétique a une bobine (50) locale et un dispositif (22) de réception et le dispositif (70) de transmission produit une liaison de signal entre la bobine (50) locale et le dispositif (22) de réception.

10. Tomodensitographe à résonance magnétique suivant la revendication 8 ou 9, dans lequel le premier signal de haute fréquence et/ou le deuxième signal de haute fréquence est l'un d'un signal de résonance magnétique, d'un signal de fréquence intermédiaire ou d'un signal d'oscillateur local ou se compose de ceux-ci en un signal combiné et le premier signal de haute fréquence a une première fréquence médiane et le deuxième signal de haute fréquence une deuxième fréquence médiane, la première fréquence médiane et la deuxième fréquence médiane étant différentes.

11. Tomodensitographe à résonance magnétique suivant la revendication 9 ou suivant la revendication 10, dans la mesure où elle dépend de la revendication 9, dans lequel le dispositif de réception a un traitement numérique du signal pour le traitement des signaux reçus par le système de transmission et le traitement numérique du signal est conçu pour compenser, au moins en partie, une diaphonie entre les signaux.

12. Procédé de transmission d'un premier signal de haute fréquence et d'un deuxième signal de haute fréquence pour un tomodensitographe (1) à résonance magnétique, par un système (70) de transmission suivant la revendication 1, dans lequel, dans un stade du procédé, on envoie le premier signal de haute fréquence à la première entrée (81) du signal du premier étage d'attaque (80) de signal et on l'achemine à la première sortie (82) du signal sur le premier brin (73), on envoie le deuxième signal de haute fréquence à la deuxième entrée (86) du signal du deuxième étage d'attaque (85) de signal et on l'achemine à la deuxième sortie (87) du signal sur le deuxième brin (74), le premier signal de haute fréquence étant indépendant du deuxième signal de haute fréquence et le premier signal de haute fréquence et le deuxième signal de haute fréquence étant transmis indépendamment l'un de l'autre à un bout de la ligne (71) de transmission éloigné du premier étage d'attaque (80) de signal et du deuxième étage d'attaque (85) de signal.
